(19) Europäisches Patentamt
European Patent Office
Office européen des brevets

(11) Publication number: **0 387 895 A2**

(12) **EUROPEAN PATENT APPLICATION**

(21) Application number: **90105001.3**

(22) Date of filing: **16.03.90**

(51) Int. Cl.5: **C07F 7/10, C08G 69/18, C07D 205/08**

(30) Priority: **16.03.89 BG 87681/89**

(43) Date of publication of application: **19.09.90 Bulletin 90/38**

(84) Designated Contracting States: **AT CH DE DK FR IT LI NL**

(71) Applicant: **N I S PRI VCHTI**
**Boul. Kl. Ohridski 8**
**Sofia(BG)**

(72) Inventor: **Mateva, Rosa Petrova**
**ul. Ginzi 18**
**Sofia(BG)**
Inventor: **Delev, Ognjan Nikolov**
**Komplex Druzba-2, Blok 419-A**
**Sofia(BG)**
Inventor: **Dimanov, Stanislav Pavlov**
**Komplex Mladost Blok 427-A**
**Sofia(BG)**
Inventor: **Nescheva, Gulinka Dimitrova**
**Komplex Ljulin, Blok 809-A**
**Sofia(BG)**

(74) Representative: **von Füner, Alexander, Dr. et al**
**Patentanwälte v. Füner, Ebbinghaus, Finck**
**Mariahilfplatz 2 & 3**
**D-8000 München 90(DE)**

# (54) N-substituted lactam derivatives as activators and co-monomers for the activated anionic polymerisation of lactams.

(57) Die Erfindung betrifft N-substituierte Lactamderivate, die als Aktivatoren und Comonomeren bei der aktivierten anionischen Polymerisation von Lactamen eingesetzt werden können. Diese Verbindungen sind stabil, nicht toxisch, leicht dosierbar, erhöhen die Polymerisationsgeschwindigkeit und bewirken den Erhalt eines Produktes mit besseren physikalisch-mechanischen Eigenschaften. Die erhaltenen Homo- und Copolyamide werden im Maschinenbau, im Eisenbahntransportwesen, in der Landwirtschaft, in der Leicht- und in der Nahrungsmittelindustrie angewendet.

EP 0 387 895 A2

# N-SUBSTITUIERTE LACTAMDERIVATE ALS AKTIVATOREN UND COMONOMEREN DER AKTIVIERTEN ANIONISCHEN POLYMERISATION VON LACTAMEN

Die Erfindung betrifft N-substituierte Lactamderivate, die als Aktivatoren und Comonomeren der aktivierten anionischen Polymerisation von Lactamen eingesetzt werden können. Die erhaltenen Homo- und Copolyamide besitzen gute physikalisch-mechanische Eigenschaften und werden im Maschinenbau, im Eisenbahntransportwesen, in der Landwirtschaft, in der Leicht- und Nahrungsmittelindustrie eingesetzt.

Bekannt ist, daß N-Acetylcaprolactam (AcCl) breite Anwendung in der Industrie als Aktivator der anionischen Polymerisation von Lactamen findet (US-PS 3 621 001; J. Stehli Č ek, J. Labsky, J. Šebenda, Coll.Czech.Chem.Commun., 32, 545, 1967). Das Lactam ist jedoch gegen Luftfeuchtigkeit und Licht stark empfindlich. Die bei der Zersetzung freigewordene Essigsäure vermindert sowohl seine Aktivität, als auch die mittlere Molekülmasse des Polymers und verschlechtert zudem die Molekülmassenverteilung. Es ist dunkel verfärbt und besitzt schlechte physikalisch-mechanische Eigenschaften.

Gegenstand der Erfindung sind neue N-substituierte Lactamderivate, die bei der aktivierten anionischen Polymeri sation von Lactamen als Aktivatoren und Comonomeren verwendet werden können. Sie dürfen nicht toxisch sein, um sicher gelagert zu werden; sie müssen ferner leicht dosierbar sein, die Polymerisationsgeschwindigkeit erhöhen und die Herstellung von Produkten mit optimaler Molekülmasse sowie einen hohen Umwandlungsgrad des Monomeren mit verbesserten physikalisch-mechanischen Eigenschaften ermöglichen.

Diese Aufgabe der Erfindung kann durch N-substituierte Lactamderivate der allgemeinen Formel

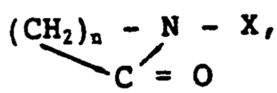

worin n = 5 bis 12, gelöst werden, wobei
X = $-Si(R)_p(R_1)_q$; p = 2, 3; q = 0, 1;
R = $-CH_3$, $-C_2H_5$; $R_1$ = $-C_6H_5$; I

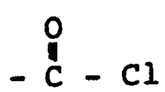 II

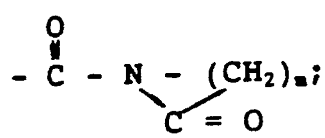 m ≠ n; m = 5 bis 12 III

bedeuten.

Die Mengen betragen 0,2 bis 2 mol%, die in Kombination mit Initiatoren wie Na,

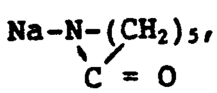

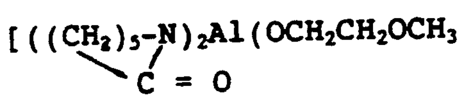 und $[H_2Al(OCH_2CH_2OCH_3)_2]\cdot Na$

als Aktivatoren der aktivierten anionischen Polymerisation von Lactamen der allgemeinen Formel

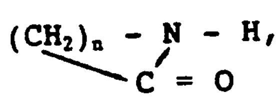

worin n = 5 bis 12,
verwendet werden können.

Die Verbindungen vom Typ I sind in dosierten Mengen über 2 mol% gegenüber dem Ausgangslactam

außer als Aktivatoren auch als Comonomere einsetzbar, wodurch verschiedene Copolyamide hergestellt werden können.

Die Eigenschaften der in Anwesenheit der erfindungsgemäßen Aktivatoren erhaltenen Produkte sind in den Tabellen 1 und 2 sowie in Abb. 1 dargestellt und mit den in Anwesenheit von AcCl erhaltenen verglichen. Die Aktivatoren I und II übertreffen AcCl im

- Umwandlungsgrad des Monomeren und in der
- mittleren Molekülmasse,

die Aktivatoren I und III erhöhen dagegen die Thermostabilität.

Ein besonderer Vorteil der Aktivatoren vom Typ I ist ihr Einfluß auf die Molekülmassenverteilung (MMV)(Abb.1). Die Anwendung von AcCl führt zum Erhalt eines Polymeren mit höherem Polydispersionsgrad (Kurve 2).

Die physikalisch-mechanischen Eigenschaften der Polyamidproben 6 (PA-6), Polyamid (PA-12), hergestellt in Anwesenheit der erfindungsgemäßen Aktivatoren sind aus Tabelle 2 ersichtlich. Sie wurden mit den gemäß BP 1 515 902 erhaltenen verglichen.

Die in Anwesenheit von verschiedenen Mengen von I erhaltenen Copolyamide besitzen eine Reihe von Vorteilen im Vergleich zu den Homopolyamiden, und zwar:

- höhere Schlagfestigkeit,
- niedrigere Wasser- und Feuchtigkeitsaufnahme, sowie
- größere Stabilität der Produkte bei ihrer Anwendung.

Die erfindungsgemäßen Copolyamide sind hochmolekulare Verbindungen der allgemeinen Formel

$$\ldots\text{-NH-}(CH_2)_n\text{-CO-}\underset{X}{N}\text{-}(CH_2)_n\text{-CO-}\ldots$$

wobei $n = 5$ bis $12$; $X = Si(R)_p(R_1)_q$ $p = 2, 3$; $q = 0, 1$;
$R = -CH_3, -C_2H_5$, $R_1 = -C_6H_5$
sind.

Mit der Erhöhung der Comonomerenmenge des Typs I verändern sich eine Reihe physikalischer Konstanten der Copolyamide. Die Löslichkeit sinkt stark bei niedrigen Temperaturen. Bei 10% Trimethylsilylcaprolactam (SL) im Polymerisationsgemisch entsteht ein in konzentrierter Schwefelsäure, m-Cresol und Dimethylsulfoxid unlösliches Copolyamid. Der Schmelzpunkt und der Kristallinitätsgrad sinken mit erhöhtem Anteil der Comonomeren. In Tabelle 3 wird die Veränderung dieser Größen für PA-12 mit verschiedener Zusammensetzung wiedergegeben (SL).

Mit der Erhöhung des Comonomergehalts vermindert sich die Wasseraufnahme des Copolymers (Tabelle 4). Die Angaben beziehen sich auf das Copolymer von ε-Caprolactam und SL.

Die physikalisch-mechanischen Eigenschaften der Copolyamidproben tendieren zur Erhöhung der Zug-, Biege- und Schlagfestigkeit und zur Verhinderung der relativen Zugverlängerung. Daraus ergibt sich die Möglichkeit, verschiedene Konstruktionsdetails auf ihrer Basis herzustellen. In Tabelle 5 sind die Ergebnisse der physikalisch-mechanischen Prüfungen der Proben aus PA-6 mit 5%igem SL-Gehalt dargestellt.

Die neuen erfindungsgemäßen Verbindungen werden in an sich bekannter Weise hergestellt. Es können die üblichen, bei der Herstellung von N-substituierten Lactamen vorgesehenen Synthesen durchgeführt werden (siehe BG-Urheberschein 44953).

Die erfindungsgemäßen Verbindungen sollen nachfolgend anhand von Beispielen erläutert werden.

<u>Beispiel 1</u>

ω- Dodekalactam wird in einem Reaktionsgefäß unter inerter Atmosphäre geschmolzen, die Schmelze bis zu 160 °C temperiert, wonach unter ständigem Rühren 1 mol% DL und nach weiteren 3 Minuten 1 mol% SL hinzugefügt wird. Das Gemisch wird gut verrührt und in Matritzen mit der Form des Produktes eingegossen. Die Polymerisation verläuft während zwei Stunden bei einer Anfangstemperatur von 220 °C und einer Endtemperatur von 160 °C.

<u>Beispiel 2</u>

Man verfährt wie in Beispiel 1, jedoch wird ε-Caprolactam als Monomer zugesetzt. Der Initiator DL und der Aktivator SL werden bei 135 °C zugefügt. Die Anfangstemperatur der Polymerisation beträgt 180 °C, die Endtemperatur 140 °C; die Dauer der Reaktion beträgt 2 Stunden.

Beispiel 3

Man verfährt ähnlich wie in Beispiel 1, Initiator ist jedoch $[H_2Al(OCH_2CH_2OCH_3)_2]^{\bullet}Na$ in einer Menge von 1 mol%.

Beispiel 4

Man verfährt ähnlich wie in Beispiel 2, Initiator ist der in Beispiel 3 angegebene.

Beispiel 5

Man verfährt ähnlich wie in Beispiel 1, als Initiator verwendet man jedoch Na-Caprolactamat.

Beispiel 6

Man verfährt ähnlich wie in Beispiel 2, als Initiator verwendet man jedoch den in Beispiel 5 angegebenen.

Beispiel 7

Man verfährt ähnlich wie in Beispiel 1, als Aktivator verwendet man jedoch N-Caprolactam-N′-dodekalactamcarbonyl in einer Menge von 0,5 mol%.

Beispiel 8

Man verfährt ähnlich wie in Beispiel 2, als Aktivator verwendet man jedoch den in Beispiel 7 angegebenen.

Beispiel 9

Man verfährt ähnlich wie in Beispiel 1, als Aktivator verwendet man jedoch Caprolactamcarbonylchlorid in einer Menge von 1 mol%.

Beispiel 10

Man verfährt ähnlich wie in Beispiel 2, Aktivator ist jedoch der in Beispiel 9 angegebene.

Beispiel 11

Man verfährt ähnlich wie in Beispiel 2, Aktivator ist jedoch Dimethylphenylsilylcaprolactam.

Beispiel 12

In einem Reaktionsgefäß wird ω-Dodekalactam geschmolzen und danach die Temperatur auf 160 °C erhöht. Der Initiator wird in einer Menge von 1 mol% und das Comonomer SL in einer Menge von 10 mol% zugefügt. Das Gemisch wird homogenisiert und in Matrizen in Form des Erzeugnisses verteilt. Die Polymerisation verläuft bei 190 °C während 2 h. Das nach diesem Verfahren erhaltene Blockpolyamid ist ein unlösliches Produkt mit einer Wasseraufnahmefähigkeit von 0,4%.

Beispiel 13

Man verfährt ähnlich wie in Beispiel 12, als Monomer wird jedoch ε-Caprolactam verwendet. Das Monomer schmilzt bei 135 °C und wird daraufhin mit den in Beispiel 12 angeführten Zugaben homogenisiert. Das Polymerisationsgemisch wird in Matrizen gegossen, wo es im Laufe von 2 h bei 150 °C polymerisiert. Das entstandene unlösliche Produkt hat eine Wasseraufnahmefähigkeit von 2,2%.

Beispiel 14

Man verfährt ähnlich wie in Beispiel 13, als Comonomer wird SL in einer Menge von 5% verwendet.

Beispiel 15

Man verfährt ähnlich wie in Beispiel 13, als Comonomer verwendet man Dimethylphenylsilylcaprolactam in einer Menge von 20% im Verhältnis zum Monomer. Die Polymerisation verläuft bei 180 °C während 2,5 h. Es entsteht ein unlösliches Produkt.

Tabelle 1

| Monomer | Katalytisches System | Viskosimetrische Molekülmasse | Ausbeute | Destruktionstemperaturindex °C 5% | Destruktionstemperaturindex °C 50% |
|---|---|---|---|---|---|
| ω-Caprolactam | Na/AcCL | 58 000 | 92,4 | 350 | 435 |
| | NaCL/ - | 32 400 | 21,8 | 320 | 420 |
| | NaCL/SL | 85 200 | 97,3 | 370 | 460 |
| | DL/AcCL | 55 700 | 93,6 | 350 | 440 |
| | DL/COCl | 67 400 | 95,1 | 365 | 430 |
| | DL/SL | 63 600 | 95,9 | 380 | 460 |
| | DL/CL-CO-DDL | 74 350 | 95,9 | 395 | 470 |
| ε-Caprolactam | Na/AcCL | 18 800 | 93,1 | 345 | 430 |
| | NaCL/- | 10 500 | 24 | 315 | 420 |
| | NaCL/SL | 21 200 | 95,2 | 345 | 460 |
| | DL/- | 16 300 | 60 | 350 | 435 |
| | DL/AcCL | 18 500 | 94,5 | 340 | 430 |
| | DL/COCl | 19 800 | 93,5 | 345 | 430 |
| | DL/SL | 23 800 | 96,5 | 360 | 460 |
| | DL/CL-CO-DDL | 23 200 | 96 | 380 | 475 |

**Bemerkungen zu Tabelle 1:** Polymerisationsdauer: 30 min

**Benennungen:**

AcCL: 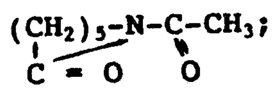

COCL: 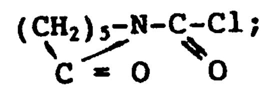

NaCL: 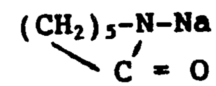

SL: 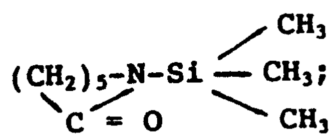

DL: 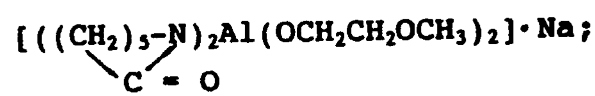

CL-CO-DDL: 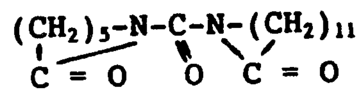

Tabelle 2

| Physikalisch-mechanische Eigenschaften | Proben erhalten gemäß Beispiel Nr. | | | | | | | | | | | Vergleichsproben | |
|---|---|---|---|---|---|---|---|---|---|---|---|---|---|
| | I | II | III | IV | V | VI | VII | VIII | IX | X | XI | PA-12 | PA-6 |
| Zugfestigkeit, MPa | 68-83 | 68-80 | 65-85 | 68-76 | 65-72 | 68-90 | 50-63 | 48-65 | 65-75 | 65-90 | 72-87 | 48-50 | 64-75 |
| relative Zugverlängerung, % | 20-25 | 8-10 | 18-25 | 6-9 | 18-23 | 8-10 | 10-17 | 10-17 | 15-20 | 8-9 | 9-12 | 15-20,5 | 4-4,5 |
| Stärke nach Brinell, MPa | 86-98 | 78-124 | 85-95 | 80-112 | 78-92 | 73-95 | 83-92 | 83-95 | 90-110 | 90-120 | 67-100 | 83-98 | 72-128 |
| Biegefestigkeit, MPa | 70-95 | 50-90 | 65-85 | 55-92 | 65-90 | 52-85 | 45-67 | 45-86 | 65-85 | 65-85 | 60-95 | 50-90 | 42-65 |
| Elastizitätsmodul, MPa | 1800-2000 | 1600-2500 | 1850-2100 | 1570-2200 | 1600-2000 | 1600-2300 | 1450-1900 | 1430-1875 | 1500-2000 | 1300-2000 | 1800-2500 | 1500-1800 | 1400-2500 |
| Schlagfestigkeit, $kJ/m^2$ | 45-55 | 20-30 | 45-50 | 18-25 | 45-55 | 15-25 | 45-60 | 15-20 | 50-60 | 10-15 | 40-60 | 45-55 | 6-25 |
| Dichte, $g/cm^3$ | 1,04 | 1,10 | 1,03 | 1,11 | 1,04 | 1,15 | 1,05 | 1,12 | 1,06 | 1,17 | 1,13 | 1,03 | 1,14 |

* Gemäß BP 1 515 902.

Tabelle 3

| Gehalt an Comonomer | Schmelzpunkt | Kristallinitätsgrad |
|---|---|---|
| 5% | 164 °C | 73% |
| 10% | 151 °C | 69% |
| 20% | 149 °C | 66% |

Tabelle 4

| | | | | |
|---|---|---|---|---|
| Comonomergehalt, % | 2 | 5 | 10 | 20 |
| Wasseraufnahme, % | 8,5 | 4,1 | 2,2 | 2,0 |

Tabelle 5

| Eigenschaften | Maß | erfindungsgemäß | gemäß BP 1 515 902 |
|---|---|---|---|
| 1. Zugfestigkeit | MPa | 65- 70 | 40- 62 |
| 2. Biegefestigkeit | MPa | 90-110 | 42- 90 |
| 3. Schlagfestigkeit | kJ/m$^2$ | 30- 40 | 6- 25 |
| 4. relative Zugverlängerung | % | 25- 30 | 70-250 |

## Claims

1. N-substituierte Lactamderivate der allgemeinen Formel

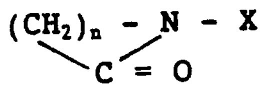

worin n = 5 bis 12,
X = $-Si(R)_p(R_1)_q$ p = 2, 3; q = 0, 1;
R = $CH_3$, $-C_2H_5$; $R_1$ = $C_6H_5$; I

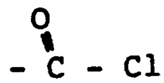 II

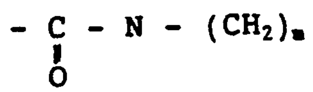 m ≠ n, m = 5 bis 12 III

bedeuten.

2. Verwendung der N-substituierten Lactame gemäß Anspruch 1 als Aktivatoren der anionischen Polymerisation.

3. Verfahren zur anionischen Polymerisation von Lactamen der allgemeinen Formel

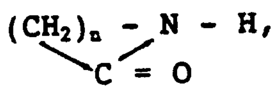 n = 5 bis 12;

**dadurch gekennzeichnet,** daß man als Aktivatoren Verbindungen gemäß Anspruch 1 in Mengen von 0,2 bis 2.mol% gegenüber dem Monomer bei einer Temperatur von 140 bis 220 °C in Kombination mit Initiatoren Na,

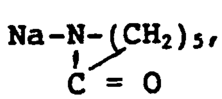 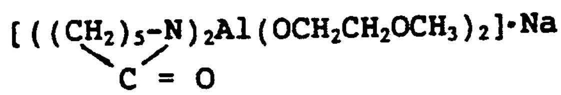

und $[H_2Al(OCH_2CH_2OCH_3)_2]^{\bullet}Na$ verwendet.

4. Verwendung von N-substituierten Lactamderivaten des Typs I gemäß Anspruch 1 in einer Menge von 21 bis 50 mol% als Comonomere bei der Polymerisation von Lactamen.

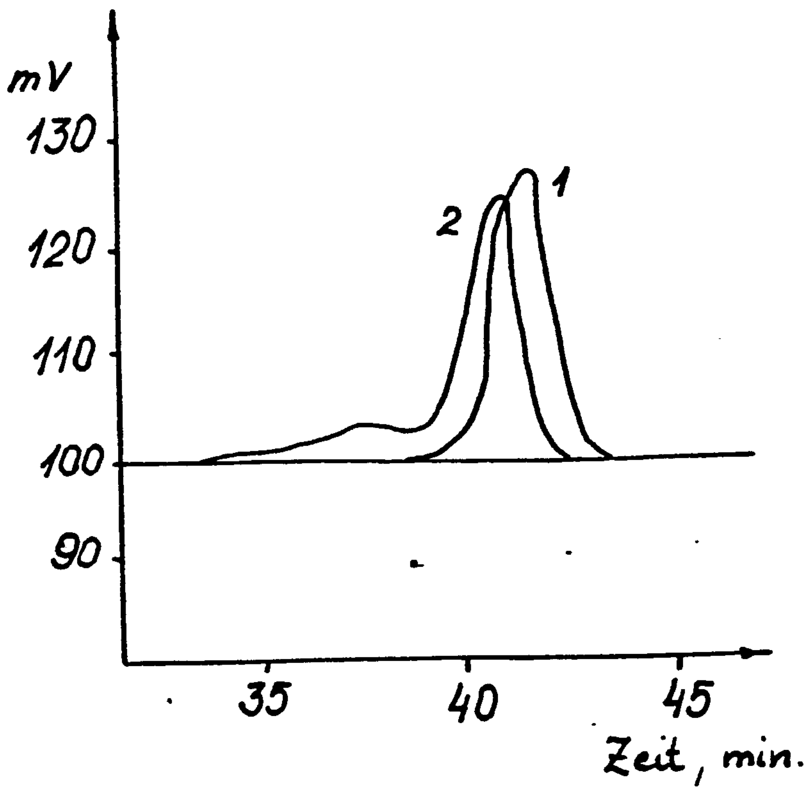


Abb. 1: Molekülmassenverteilung von PA-6, erhalten in Anwesenheit von DL/SL (Kurve 1) und DL/AcCL (Kurve 2)